## Europäisches Patentamt

## European Patent Office

## Office européen des brevets

(19)

(11) Veröffentlichungsnummer: **0 258 521**
**B1**

(12)

# EUROPÄISCHE PATENTSCHRIFT

(45) Veröffentlichungstag der Patentschrift:
02.05.90

(51) Int. Cl.⁴: **A61M 35/00**

(21) Anmeldenummer: 87102306.5

(22) Anmeldetag: 18.02.87

(54) Mittel zur Applikation von transdermal resorbierbaren Wirkstoffen.

(30) Priorität: 23.08.86 DE 3628688
30.08.86 DE 3629565

(43) Veröffentlichungstag der Anmeldung:
09.03.88 Patentblatt 88/10

(45) Bekanntmachung des Hinweises auf die Patenterteilung:
02.05.90 Patentblatt 90/18

(84) Benannte Vertragsstaaten:
AT BE CH DE ES FR GB GR IT LI LU NL SE

(56) Entgegenhaltungen:
EP-A- 0 136 393
WO-A-81/03423
DE-A- 2 301 821
DE-A- 2 722 273
FR-A- 2 561 505
US-A- 3 777 754
US-A- 4 230 105

(73) Patentinhaber: Latzke, Arno Walter, Mühltobel 946,
Ch-9429 Zelg/Wolfhalden(CH)
Patentinhaber: Latzke, Ralf, Mühltobel 946,
CH-9429 Zelg/Wolfhalden(CH)
Patentinhaber: Latzke, Gert, Mühltobel 946,
CH-9429 Zelg/Wolfhalden(CH)

(72) Erfinder: Latzke, Arno Walter, Mühltobel 946,
Ch-9429 Zelg/Wolfhalden(CH)
Erfinder: Latzke, Ralf, Mühltobel 946,
CH-9429 Zelg/Wolfhalden(CH)
Erfinder: Latzke, Gert, Mühltobel 946,
CH-9429 Zelg/Wolfhalden(CH)

(74) Vertreter: Werner, Hans-Karsten, Dr. et al,
Deichmannhaus am Hauptbahnhof, D-5000 Köln 1(DE)

## Beschreibung

Gegenstand der vorliegenden Erfindung ist ein Mittel zur Applikation von transdermal resorbierbaren Wirkstoffen, welches eine intensive und langanhaltenden Einwirkung der transdermal resorbierbaren Wirkstoffe gestattet und deren Wirksamkeit durch gleichzeitige Einwirkung von Wärme steigert.

Eine Reihe von Schmerzen, Verkrampfungen und krampfhaften Störungen beruhen auf einer ungenügenden oder ungleichmäßigen Durchblutung der Außenhaut. In vielen Fällen läßt sich durch gleichmäßige Einwirkung von Wärme Linderung, Besserung oder sogar Heilung erzielen. Die Behandlung mit Infrarotstrahlen, Wärmeflaschen, Heizkissen, heißen Packungen und Umschlägen ist stets mit erheblichem Aufwand verbunden und daher nicht immer und ohne weiteres durchführbar.

Weiterhin ist bekannt, daß transdermal, resorbierbare Wirkstoffe, insbesondere Heilpflanzenöle, besonders dann therapeutisch wirksam sind, wenn sie ausreichend lange einwirken können und die Möglichkeit haben, in die Haut einzudringen. Es wird bereits seit langem nach Möglichkeiten gesucht, die Wirksamkeit der transdermal resorbierbaren Wirkstoffe zu steigern.

Aus der EP-A 0 163 045 sind Mittel zur Wärmeisolierung sowie zur Speicherung und Verteilung von Wärme, insbesondere von Körperwärme, auf Flächen der Außenhaut bekannt, welche aus einem Laminat, einer Schaumstoffschicht, einer flexiblen, wärmeleitenden Metallschicht und einer weiteren Schaumstoffschicht bestehen. Diese Mittel haben sich bereits als wärmende Pflaster, Kissen, Matten etc. bewährt.

Die Erfindung hat sich die Aufgabe gestellt, dieses Prinzip der Therapie mit Wärme zu kombinieren mit dem Prinzip der Anwendung von transdermal resorbierbaren Wirkstoffen, insbesondere Heilpflanzenölen, um dadurch die therapeutische Wirksamkeit zu steigern und zu optimieren.

Diese Aufgabe kann überraschend gut und einfach gelöst werden durch Mittel zur Applikation von transdermal resorbierbaren Wirkstoffen, bestehend aus mindestens einem auswechselbaren, mit den transdermal resorbierbaren Wirkstoffen getränkten Träger (1) und einer Halterung (2) für den Träger, bestehend aus einem Laminat aus

a) einer 1 bis 5 mm starken, geschlossenporigen Polyethylenschaumstoffschicht (3),

b) einer flexiblen, wärmeleitfähigen Metallfolie (4),

c) einer 1 bis 4 mm starken, gegebenenfalls gefärbten geschlossenporigen Polyethylenschaumstoffschicht (5) und

d) einer 1 bis 4 mm starken, gegebenenfalls genoppten, geschlossenporigen Polyethylenschaumstoffschicht (6), aus welcher mindestens ein vorzugsweise gezacktes Stück (7) ausgestanzt ist, wobei das Stück (7) flächenmäßig gleich groß oder geringfügig größer ist als den Träger (1),
oder

a) einer 2 bis 5 mm starken, geschlossenporigen Polyethylenschaumstoffschicht (3),

b) einer flexiblen, wärmeleitfähigen Metallfolie (4),

c) einer 2 bis 5 mm starken, gegebenenfalls genoppten, geschlossenporigen Polyethylenschaumstoffschicht (6), aus welcher mindestens ein vorzugsweise gezacktes Stück (7) ausgestanzt ist, wobei das Stück (7) flächenmäßig gleich groß oder geringfügig größer ist als der Träger (1)
oder

a) einer 2 bis 6 mm starken, geschlossenporigen Polyethylenschaumstoffschicht (3), die gegebenenfalls gefärbt ist, und

b) einer 2 bis 5 mm starken, gegebenenfalls genoppten, geschlossenporigen Polyethylenschaumstoffschicht (6), aus welcher mindestens ein vorzugsweise gezacktes Stück (7) ausgestanzt ist, wobei dann der saugfähige Träger (1) einseitig mit der flexiblen, wärmeleitfähigen Metallfolie (4) fest verbunden ist.

Diese Mittel weisen zur leichteren Befestigung auf dem Körper vorzugsweise auf der Schaumstoffschicht (3) eine Gewebeschicht (8) auf, die vorzugsweise aus Borschednylon besteht. Diese Gewebeschicht gestattet die Befestigung der erfindungsgemäßen Mittel auf dem Körper mit Hilfe von hautverträglichen, selbstklebenden Pflastern oder aber elastischen Binden mit Klettverschlüssen.

Die erfindungsgemäßen Mittel können dann in Form von Pflastern, aber auch Stirnbändern oder Kissen hergestellt werden, wobei Stirnbänder oder Kissen meist mehrere ausgestanzte Stücke (7) und darin auswechselbar eingelegte, mit transdermal resorbierbaren Wirkstoffen getränkte Träger (1) aufweisen.

Als Träger (1) können vorzugsweise Vliese und insbesondere alle hautverträglichen Wirrfaservliese verwendet werden, die eine ausreichende Steifigkeit aufweisen und mit den transdermal resorbierbaren Wirkstoffen getränkt werden können. Prinzipiell kommen aber auch andere ausreichend steife, hautverträgliche, gegen transdermal resorbierbare Wirkstoffe beständige, saugfähige Träger, wie offenporige Schaumstoffe, Stoffe etc., in Frage. Die Form dieser getränkten Träger kann beliebig gewählt werden, beispielsweise quadratisch, rechteckig oder vieleckig. Besonders einfach und bevorzugt ist die kreisrunde Form. Diese mit transdermal resorbierbaren Wirkstoffen getränkten Träger können entweder vom Anwender selbst frisch zubereitet werden, oder aber vom Hersteller - ähnlich Erfrischungstüchern - eingesiegelt mitgeliefert werden.

Die ausgestanzten Stücke (7) sind flächenmäßig gleich groß oder geringfügig größer als der Träger (1) und dabei vorzugsweise gezackt. Diese Ausgestaltungsform sorgt einerseits für eine gute Halterung des Trägers in der Ausstanzung und andererseits zur Ausbildung von kleinen Luftkammern am Rande des Trägers, in denen sich Dämpfe der transdermal resorbierbaren Wirkstoffe ansammeln können.

Die geschlossenporigen Polyethylenschaumstoffschichten (3), (5) und (6) können in dem genannten Rahmen zwischen 1 und 5 bzw. 1 und 6 mm Stärke schwanken, insgesamt sollte das Gesamtlaminat jedoch Stärken aufweisen zwischen 5 und 12, vorzugsweise 6 bis 10 mm. Dünnere Laminate sind schwierig herzustellen und sorgen nicht mehr für eine ausreichende Speicherung der Wärme. Stärkere Laminate werden zu steif und unhandlich und tragen zu stark auf.

Als flexible wärmeleitfähige Metallfolie wird insbesondere Aluminiumfolie eingesetzt, die vorzugsweise Stärken zwischen 20 und 50 µm aufweist.

Bei einer einfacheren und fertigungstechnisch bevorzugten Ausführungsform entfällt die Schaumstoffschicht (5), so daß der Träger (1) unmittelbar mit der Metallfolie (4) in Berührung kommt. Dies kann sogar zu einer noch schnelleren und stärkeren Erwärmung des Trägers (1) führen und somit zu einem schnelleren und stärkeren Wirkungseintritt.

Schließlich ist es auch möglich, die Metallfolie (4) in dem Laminat der Halterung (2) ganz wegzulassen, wenn der saugfähige Träger (1) einseitig mit dieser flexiblen, wärmeleitfähigen Metallfolie (4) fest verbunden ist. Bei dieser Ausführungsform sind vorzugsweise die beiden verbleibenden Schaumstoffschichten (3) und (6) verschiedenfarbig eingefärbt. Weiterhin wird bei dieser Ausführungsform insbesondere die Schicht (3) dicker gewählt und/oder aus einem gröberporigen Schaumstoff gewählt, der ein höheres Wärmeisoliervermögen hat.

Die erstaunlich gute Wirksamkeit der erfindungsgemäßen Mittel ist auf folgenden Wirkmechanismus zurückzuführen: das Gesamtlaminat der Halterung (3) führt wie bei den Mitteln gemäß EP-A 0 163 045 zu einer guten Speicherung und Verteilung der Körperwärme. Dabei zeigt sich, daß die Rückführung der Wärme zum Körper besonders stark ist in dem ausgestanzten Stück (7). Genau an dieser Stelle liegt aber der mit den transdermal resorbierbaren Wirkstoffen getränkte Träger (1), so daß die sich darin befindlichen transdermal resorbierbaren Wirkstoffe besonders stark erwärmt werden. Dies führt zu einem erhöhten Dampfdruck der transdermal resorbierbaren Wirkstoffe und einer verstärkten Wirksamkeit im Bereich der ausgestanzten Stücke (7). In den Luftkammern am Rande des Trägers können sich diese Dämpfe der transdermal resorbierbaren Wirkstoffe sammeln, so daß sie über längere Zeit ihre Wirksamkeit entfalten können. Die Metallfolie (4) wirkt dabei gleichzeitig als Dampfsperre und unterbindet ein unerwünschtes vorzeitiges Entweichen der Dämpfe durch den Schaumstoff.

Dadurch daß die an die Haut angrenzende Schaumstoffschicht (6) vorzugsweise genoppt ist, findet eine gewisse Be- und Entlüftung statt und wird eine unerwünschte Schweißbildung unterdrückt. Durch diese Noppung kann auch ein gewisser Anteil der transdermal resorbierbaren Wirkstoffdämpfe über den Bereich der Ausstanzung hinaus wirksam werden. Die Noppung besteht daher vorzugsweise aus einem quadratischen Muster von eingeprägten Rillen im Abstand von 1 bis 3 mm. Die Rillen sind ca. 0,5 bis 2 mm tief. Statt der Noppung ist es auch möglich, die Schicht (6) mit einer Gewebeschicht zu laminieren.

Messungen der Hauttemperatur unter Einwirkung der erfindungsgemäßen Mittel haben ergeben, daß die stärkste Erwärmung am Rande des Trägers (1), nämlich in den Belüftungskammern, erfolgt. Dies kann nur dadurch erklärt werden, daß an diesen Stellen die geringste Wärmeisolierung besteht, so daß die rundherum von der Halterung (2) gesammelte Wärme relativ ungestört an die Haut zurückgeführt werden kann und an diesen Stellen die Dämpfe der transdermal resorbierbaren Wirkstoffe in relativ hoher Konzentration auf die Haut einwirken können.

Bei der Herstellung der Halterung (2) bestünde zwar prinzipiell die Möglichkeit, die Polyethylenschaumstoffschichten (5) und (6) bzw. (4) und (6) oder gar (3) und (6) jeweils aus einem Stück herzustellen, und aus diesem Stück dann das Stück (7) in der Weise auszustanzen, daß noch bis zu 4 mm Schaumstoffschicht zwischen dem Träger (1) und der Metallfolie (4) verbleiben können. Dies ist jedoch fertigungstechnisch wesentlich schwieriger als die jeweiligen beiden Schichten, wie oben angegeben, aus Teilschichten miteinander zu laminieren. Dies hat den weiteren Vorteil, daß die Schaumstoffschichten (5) bzw. (3) eingefärbt werden können, so daß das ausgestanzte Stück (7) optisch deutlich ins Auge fällt. Dies erleichtert die Einsetzung des Trägers (1) in die Ausstanzung. Statt das Stück (7) auszustanzen ist es natürlich auch möglich, dies heiß einzuprägen, wobei jedoch kaum scharfe Zacken erhältlich sind. Zur besseren Befestigung des Trägers (1) an die Halterung (2) können dann auch ein Adhäsionskleber oder ein Stück doppelseitige Klebefolie verwendet werden.

Die Träger (1) werden meist nach der Anwendung verworfen, während die Halterung (2) häufig wiederverwendet werden kann. Sie kann auch bei Temperaturen bis zu 30°C gewaschen und dadurch hygienisch gehalten werden. Als in der Wärme transdermal resorbierbare Wirkstoffe kommen insbesondere Flüssigkeiten wie Öle, aber auch Tabletten, Pasten und Pulver in Frage, sofern sie einen ausreichend hohen Dampfdruck aufweisen. Insbesondere kommen Heilpflanzenöle in Frage. Bevorzugt werden als Heilpflanzenöle ätherische Öle wie Kampferöl, Grünöl, Lavendelöl, Pfefferminzöl, Rosmarinöl und Terpentinöl sowie Gemische derselben eingesetzt. Diese Öle kommen zusammen mit der langanhaltenden Erwärmung therapeutisch zum Einsatz bei Muskel- und Gelenkschmerzen, Hexenschuß, Ischias, Bandscheibenbeschwerden und rheumatischen Beschwerden. Weiterhin können sie eingesetzt werden zur Vorbeugung sowie zur Nachbehandlung von Sportschäden, Zerrungen und Prellungen. In all diesen Fällen erfolgt eine Steigerung des Gewebestoffwechsels und eine bessere Durchblutung. Die erfindungsgemäßen Mittel stellen somit einen vorzüglichen Applikator insbesondere für Heilpflanzenöle und ihre Dämpfe dar, wobei die Wirksamkeit intensiviert und verlängert wird.

Bei der Erprobung der erfindungsgemäßen Mittel hat sich gezeigt, daß insbesondere bei mehrfacher Benutzung der Halterung die Träger schlechter gehalten werden und dazu neigen, herauszufallen oder zu verrutschen. Die Befestigung des Trägers an der Halterung kann zwar prinzipiell auch durch einen Adhäsionskleber oder ein Stück doppelseitige Klebefolie erfolgen, jedoch haben sich die folgenden zusätzlichen Befestigungen besser bewährt: Sowohl an der dem Körper abgewandten Seite des Trägers (1) als auch an der dem Träger (1) zugewandten Seite der Halterung (2) wird ein Klettverschluß angebracht. Vorzugsweise wird aber an der dem Körper abgewandten Seite des Trägers (1) eine magnetisch haftende Metallplatte (9) oder ein magnetisch haftender Metalldraht (10) befestigt und in der Halterung (2) eine dem Träger (1) zugewandte Magnetfolie (11) befestigt, welche streifenförmig abwechselnd positiv und negativ dauerhaft magnetisiert ist, wobei der Abstand der magnetisierten Streifen zwischen 3 und 10 mm liegt.

Die reversible Befestigung mit Hilfe eines Klettverschlusses ist relativ einfach durchzuführen und preiswert. Der Klettverschluß hat jedoch den Nachteil, ziemlich stark aufzutragen und daher zu einer ungewollten Verdickung der erfindungsgemäßen Mittel zu führen. Diesen Nachteil wird man daher nur bei Ausführungsformen in Kauf nehmen, die eine größere Gesamtdicke des erfindungsgemäßen Mittels gestatten.

Als besonders vorteilhaft hat sich die Befestigung mit Hilfe einer magnetisch haftenden Metallplatte (9) oder eines magnetisch haftenden Metalldrahtes (10) bewährt, da diese ohne Schwierigkeiten auf der dem Körper abgewandten Seite des Trägers (1) befestigt werden können. Dies kann beispielsweise durch Einweben in das Vliesmaterial oder auch Aufkleben erfolgen. Bei der Ausführungsform, bei welcher der saugfähige Träger (1) einseitig mit der flexiblen, wärmeleitfähigen Metallfolie (4) fest verbunden ist, ist es möglich, die Metallplatte 9 oder den Metalldraht (10) zwischen dem Träger und der Metallfolie anzubringen.

Als magnetisch haftende Metallplatte oder magnetisch haftender Metalldraht kommen insbesondere Eisen, aber auch andere ferromagnetische Legierungen und Metalle in Frage. Bei Verwendung von Eisen oder Stahl kann es aus Gründen des Korrosionsschutzes sinnvoll sein, verzinkte oder sonst an der Oberfläche gegen Korrosion geschützte Eisenplatten oder Eisendrähte zu verwenden.

Als Magnetfolien, welche streifenförmig abwechselnd positiv oder negativ dauerhaft magnetisiert sind und bei denen der Abstand der magnetisierten Streifen zwischen 3 und 10 mm liegt, eignen sich handelsübliche Magnetfolien, die in Stärken zwischen 0,5 und 2 mm, vorzugsweise in Stärken zwischen 1, 0 und 1,5 mm Stärke geliefert werden, und in der gewünschten Weise abwechselnd magnetisiert sind. Die Streifen können dabei geradlinig sein oder kreisförmig, so daß die magnetisierten Streifen als konzentrische Ringe vorliegen.

Der Abstand der magnetisierten Streifen zwischen 3 und 10 mm gewährleistet eine gute Haftung der Metallplatte oder des Metalldrahtes durch magnetische Anziehung, auch wenn diese nicht unmittelbar anliegen. Dies beruht darauf, daß streifenförmig abwechselnd positiv und negativ dauerhaft magnetisierte Magnetfolien auch noch in einigen mm Abstand ein ausreichend starkes Magnetfeld erzeugen.

Die Größe der Platte (9) kann größer oder kleiner sein als die Größe der Magnetfolie (11). Vorzugsweise ist die Größe der Magnetplatte in der gleichen Größenordnung wie das ausgestanzte Stück (7). Größere Magnetfolien einzubauen, ist technisch und materialmäßig aufwendiger. Zu klein gewählte Dimensionen der Magnetfolie (11) führen dazu, daß eine ausreichende magnetische Anziehung der Metallplatte (9) oder des Metalldrahtes (10) nicht gewährleistet ist.

Prinzipiell wäre es natürlich auch möglich, die Lage der Metallplatte (9) bzw. des Metalldrahtes (10) mit der Lage der Magnetfolie (11) zu vertauschen. Dies würde jedoch bedeuten, daß der nur einmal verwendete Träger zusammen mit der relativ teuren Magnetfolie weggeworfen würde, während sie bei der beanspruchten Ausführungsform zusammen mit der Halterung (2) mehrfach verwendet werden kann.

Schließlich hat sich gezeigt, daß es in einigen Fällen vorteilhaft sein kann, bei den erfindungsgemäßen Mitteln die der Haut zugewandte Seite der Halterung (2) mit einem hautverträglichen Stoff zu überziehen. Vorzugsweise wird hierfür ein dünnes Baumwollgewebe auflaminiert oder ein anderes hautverträgliches Gewebe mit vorgefertigter Klebeschicht, wie beispielsweise das Produkt Mefix von der Firma Mölnlycke.

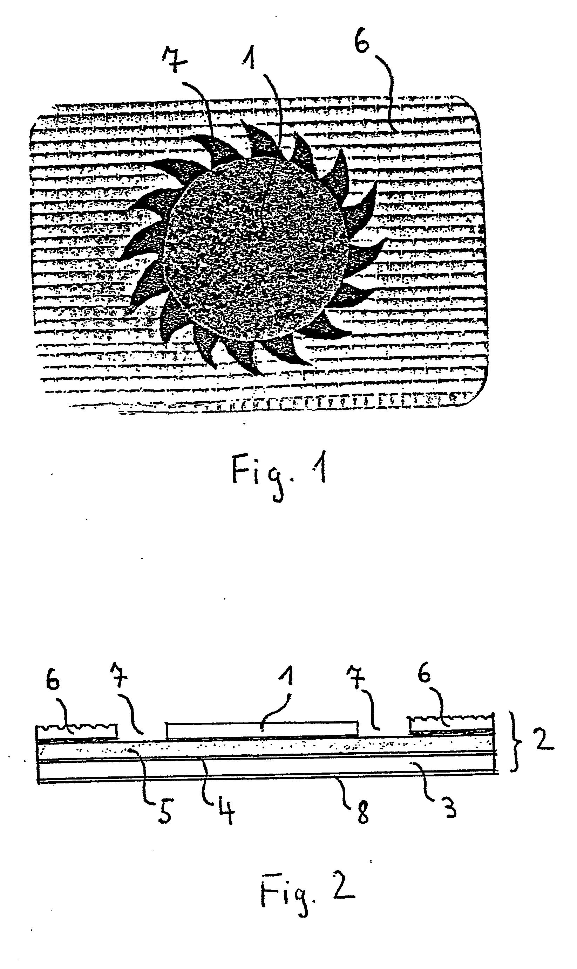

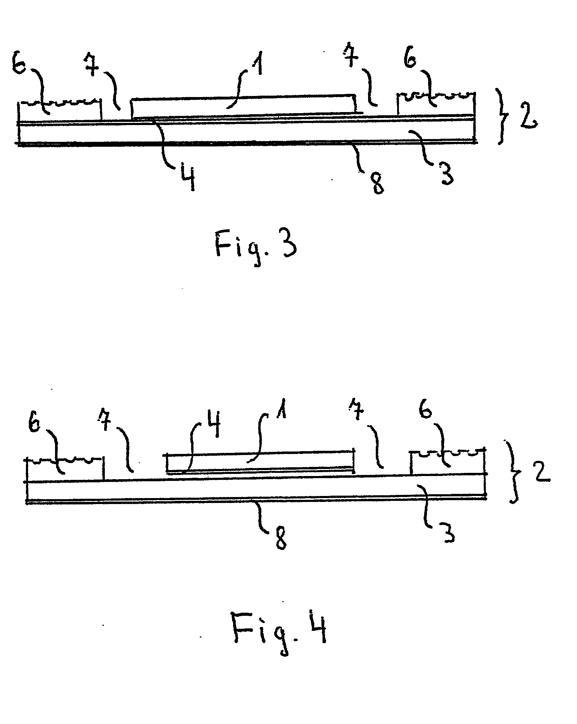

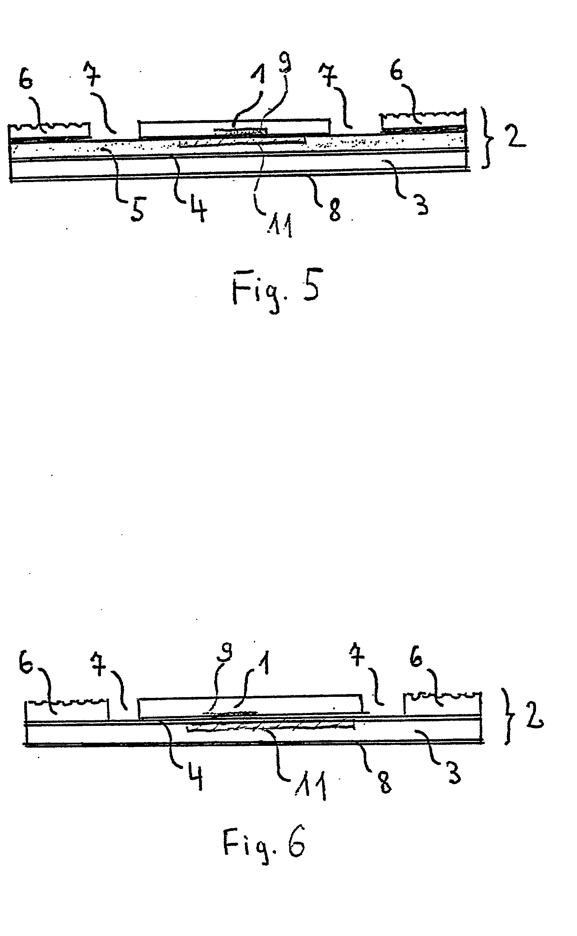

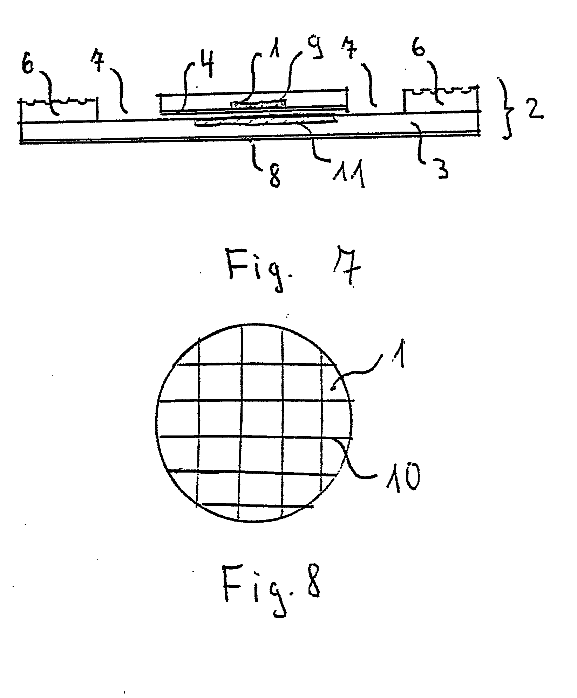

In den anliegenden Figuren sind bevorzugte Ausführungsformen des erfindungsgemäßen Mittels zur Applikation von transdermal resorbierbaren Wirkstoffen dargestellt. Fig. 1 stellt in der Aufsicht ein Mittel in der Form eines Pflasters dar. Fig. 2 zeigt den Schnitt durch dieses Mittel. Fig. 3 und 4 zeigen den Schnitt durch vereinfachte Ausführungsformen. Die Fig. 5, 6 und 7 zeigen Abwandlungen der Ausführungsformen gemäß Fig. 2, 3 und 4, bei welchen zusätzlich Metallplatten und Magnetfolien zur Befestigung des Trägers an der Halterung eingebaut sind. Fig. 8 zeigt in der Aufsicht einen Träger (1) mit einem magnetisch haftenden Metalldraht, welcher gitterförmig ausgebildet ist.

In den Figuren bedeuten jeweils

(1) einen mit den transdermal resorbierbaren Wirkstoffen getränkten Träger
(2) die gesamte Halterung
(3) eine geschlossenporige Polyethylenschaumstoffschicht
(4) eine flexible, wärmeleitfähige Metallfolie
(5) eine gegebenenfalls gefärbte, geschlossenporige Polyethylenschaumstoffschicht
(6) eine gegebenenfalls genoppte oder mit Gewebe laminierte, geschlossenporige Polyethylenschaumstoffschicht
(7) ein gezackt ausgestanztes Stück
(8) eine Gewebeschicht aus Borschednylon
(9) eine magnetisch haftende Metallplatte
(10) ein magnetisch haftender Metalldraht
(11) eine streifenförmig abwechselnd positiv und negativ dauerhaft magnetisierte Magnetfolie, wobei der Abstand der magnetisierten Streifen zwischen 3 und 10 mm liegt.

In dieser bevorzugten Ausführungsform gemäß Fig. 2 beträgt die Dicke der Polyethylenschaumstoffschicht (3) ca. 2 mm, der Polyethylenschaumstoffschicht (5) ca. 3 mm und der Polyethylenschaumstoffschicht (6) ursprünglich ebenfalls 2 mm, im genoppten Zustand 1 bis 1,8 mm. Folglich ist die Tiefe der Austanzung (7) ebenfalls ca. 1,8 mm tief. In den Ausführungsformen gemäß Fig. 3 und 4 sind die Schaumstoffschichten meist etwas stärker.

Nicht dargestellt sind das auswechselbare selbstklebende, hautverträgliche Pflaster oder die elastische Binde mit Klettverschluß zur Befestigung der erfindungsgemäßen Mittel auf der Haut. Durch die Gewebeschicht (8), die vorzugsweise aus Borschednylon besteht, haften sowohl die selbstklebenden Pflaster als auch Klettverschlüsse ausreichend fest, können jedoch ohne Beschädigung der Halterung (2) wieder entfernt werden, so daß diese Halterung (2) häufig wiederverwendet werden kann.

Das ausgestanzte Stück (7) ist gemäß Fig. 1 in der Weise gezackt, daß sägezahnförmige Belüftungskammern entstehen. Mit dem eingelegten Träger entsteht das Bild eines Blütenblattes bzw. des Strahlenkranzes einer Sonne. Prinzipiell können aber auch gerade Zacken oder sogar wellenlinienförmige und somit abgerundete Zacken zur Anwendung kommen, sofern diese in der Lage sind, den mit den transdermal resorbierbaren Wirkstoffen getränkten Träger (1) ausreichend zu halten.

Zur Herstellung eines entsprechenden Stirnbandes wird eine längliche Halterung von 5 bis 30 cm Länge und 2 bis 4 cm Breite hergestellt, in welchem nebeneinander ein oder mehrere Stücke (7) ausgestanzt sind und entsprechend kleinere Träger (1) eingelegt werden können. Für die Herstellung von Kissen werden hingegen die Dimensionen entsprechend größer gewählt und ebenfalls mehrere Stücke (7) ausgestanzt, in welche dann entsprechend große Träger (1) eingelegt werden können.

## Patentansprüche

1. Mittel zur Applikation von transdermal resorbierbaren Wirkstoffen, bestehend aus mindestens einem auswechselbaren, mit den transdermal resorbierbaren Wirkstoffen getränkten Träger (1) und einer Halterung (2) für den Träger, bestehend aus einem Laminat aus
a) einer 1 bis 5 mm starken, geschlossenporigen Polyethylenschaumstoffschicht (3),
b) einer flexiblen, wärmeleitfähigen Metallfolie (4),
c) einer 1 bis 4 mm starken, gegebenenfalls gefärbten geschlossenporigen Polyethylenschaumstoffschicht (5) und
d) einer 1 bis 4 mm starken, gegebenenfalls genoppten, geschlossenporigen Polyethylenschaumstoffschicht (6), aus welcher mindestens ein vorzugsweise gezacktes Stück (7) ausgestanzt ist, wobei das Stück (7) flächenmäßig gleich groß oder geringfügig größer ist als der Träger (1),
oder
a) einer 2 bis 5 mm starken, geschlossenporigen Polyethylenschaumstoffschicht (3),
b) einer flexiblen, wärmeleitfähigen Metallfolie (4),
c) einer 2 bis 5 mm starken, gegebenenfalls genoppten, schlossenporigen Polyethylenschaumstoffschicht (6), aus welcher mindestens ein vorzugsweise gezacktes Stück (7) ausgestanzt ist, wobei das Stück (7) flächenmäßig gleich groß oder geringfügig größer ist als der Träger (1)
oder
a) einer 2 bis 6 mm starken, geschlossenporigen Polyethylenschaumstoffschicht (3), die gegebenenfalls gefärbt ist, und
b) einer 2 bis 5 mm starken, gegebenenfalls genoppten, geschlossenporigen Polyethylenschaumstoffschicht (6), aus welcher mindestens ein vorzugsweise gezacktes Stück (7) ausgestanzt ist, wobei dann der saugfähige Träger (1) einseitig mit der flexiblen, wärmeleitfähigen Metallfolie (4) fest verbunden ist.

2. Mittel gemäß Anspruch 1, dadurch gekennzeichnet, daß auf die Schaumstoffschicht (3) eine Gewebeschicht (8) auflaminiert ist.

3. Mittel gemäß Anspruch 1 oder 2, dadurch gekennzeichnet, daß es die Form eines Pflasters, Stirnbands oder Kissens hat.

4. Mittel gemäß einem der Ansprüche 1 bis 3, dadurch gekennzeichnet, daß die der Haut zugewandten Seite der Halterung (2) mit einem hautverträglichen Stoff überzogen ist.

5. Mittel gemäß einem der Ansprüche 1 bis 4, dadurch gekennzeichnet, daß an der dem Körper abgewandten Seite des Trägers (1) eine magnetisch haftende Metallplatte (9) oder ein magnetisch haftender Metalldraht (10) befestigt ist und in der Halterung (2) eine dem Träger (1) zugewandte Magnetfolie (11) befestigt ist, welche streifenförmig abwechselnd positiv und negativ dauerhaft magnetisiert ist, wobei der Abstand der magnetisierten Streifen zwischen 3 und 10 mm liegt.

6. Mittel gemäß einem der Ansprüche 1 bis 4, dadurch gekennzeichnet, daß an der dem Körper abgewandten Seite des Trägers (1) und der dem Träger (1) zugewandten Seite der Halterung (2) ein Klettverschluß angebracht ist.

## Claims

1. Means for the application of transdermally resorbable active substances, which means consist of at least one replaceable carrier (1) impregnated with the transdermally resorbable active substances and a support (2) for the carrier consisting of a laminate comprising either.
a) a closed-cell polyethylene foam layer (3) having a thickness of 1 to 5 mm,
b) a flexible heat-conductive metal foil (4),
c) a closed-cell polyethylene foam layer (5) having a thickness of 1 to 4 mm, which may optionally be colored,
d) a closed-cell polyethylene foam layer (6) having a thickness of 1 to 4 mm, which may optionally be knopped, from which at least one portion (7), preferably in pinked shape, has been punched, the size of the area of the portion (7) being equal to or slightly larger than that of the carrier (1),
or

a) a closed-cell polyethylene foam layer (3) having a thickness of 2 to 5 mm,

b) a flexible heat-conductive metal foil (4),

c) a closed-cell polyethylene foam layer (6) having a thickness of 2 to 5 mm, which may optionally be knopped, from which at least one portion (7), preferably in pinked shape, has been punched, the size of the area of the portion (7) being equal to or slightly larger than that of the carrier (1), or

a) a closed-cell polyethylene foam layer (3) having a thickness of 2 to 6 mm, which may optionally be colored,

b) a closed-cell polyethylene foam layer (6) having a thickness of 2 to 5 mm, which may optionally be knopped, from which at least one portion (7), preferably in pinked shape, has been punched, the absorptive carrier (1) on one side being firmly bonded to a flexible heat-conductive metal foil (4).

2. The means according to claim 1, characterized in that the a fabric layer (8) is laminated onto the foam layer (3).

3. The means according to claims 1 or 2, characterized in that it is in the shape of a plaster, headband or pad.

4. The means according to anyone of claims 1 to 3, characterized in that the surface facing the skin of the support (2) is covered with a skin-compatible fabric.

5. The means according to anyone of claims 1 to 4, characterized in that a magnetically adhesive metal plate (9) or a magnetically adhesive metal wire (10) is attached to the surface remote from the skin of the carrier (1) and a magnetic foil (11) has been attached to the surface facing the carrier (1) of the support (2) which magnetic foil in stripe form has been alternatingly positively and negatively permanently magnetized, the distance of the magnetized stripes from one another being between 3 and 10 mm.

6. The means according to anyone of claims 1 to 4, characterized in that a Velcro strip fastener is attached to the surface remote from the skin of the carrier (1) and to the surface facing the carrier (1) of the support (2).

**Revendications**

1. Dispositif pour appliquer des agents aptes à se résorber à travers la peau, constitué par au moins un support (1) remplaçable, imbibé d'agents aptes à se résorber à travers la peau et, par un moyen de maintien (2) pour le support, constitué par un stratifié comprenant:

a) une couche de mousse de polyéthylène à pores fermés épaisse de 1 à 5 mm (3),

b) une feuille métallique (4) flexible et conduisant la chaleur,

c) une couche de mousse de polyéthylène (5) à pores fermés, épaisse de 1 à 4 mm, éventuellement colorée,

d) une couche de mousse de polyéthylène (6) à pores fermés, épaisse de 1 à 4 mm, le cas échéant avec des noppes, dans laquelle au moins une partie (7), de préférence dentelée, est enlevée par poinçonnage, laquelle partie (7) a une surface de grandeur égale ou légèrement supérieure à celle du support (1),

ou

a) une couche de mousse de polyéthylène (3) à pores fermés, de 2 à 5 mm d'épaisseur,

b) une couche métallique (4) flexible, conduisant la chaleur,

c) une couche de mousse de polyéthylène (6) à pores fermés épaisse de 2 à 5 mm, le cas échéant avec des noppes, dans laquelle au moins une partie (7), de préférence dentelée, est enlevée par poinçonnage, laquelle partie (7) a une surface de grandeur égale ou légèrement supérieure à celle du support (1)

ou

a) une couche de mousse de polyéthylène (6) à pores fermés, épaisse de 2 à 6 mm, qui est, le cas échéant, teintée et,

b) une couche de mousse de polyéthylène à pores fermés (6), épaisse de 2 à 5 mm, le cas échéant avec des noppes, dans laquelle une partie (7), de préférence dentelée, est enlevée par poinçonnage, tandis que le support (1) capable d'absorption est lié sur une face, de manière fixe, à la feuille métallique (4) flexible conduisant la chaleur.

2. Dispositif selon la revendication 1, caractérisé en ce qu'une couche de tissu (8) est fixée par laminage sur la couche de mousse (3).

3. Dispositif selon la revendication 1 ou 2, caractérisé en ce qu'il a la forme d'un emplâtre, d'un bandeau ou d'un coussin.

4. Dispositif selon l'une des revendications 1 à 3, caractérisé en ce que la face du moyen de maintien (2) qui est tournée vers la peau est revêtue d'un tissu toléré par la peau.

5. Dispositif selon l'une des revendications 1 à 4, caractérisé en ce qu'une plaque de métal (9) à adhérence magnétique, ou un fil métallique (10) à adhérence magnétique, est fixaée sur la face du support (1) opposée au corps, et en ce que, dans le moyen de maintien (2), est fixée une feuille aimantée (11) tournée vers le support (1) qui est magnétisée par bandes, tour à tour positivement et négativement de manière permanente, l'intervalle entre les bandes aimantées étant compris entre 3 et 10 mm;

6. Dispositif selon l'une des revendications 1 à 4, caractérisé en ce qu'une liaison auto-accrochante à boucles et crochets est disposée sur la face du support (1) opposée au corps et sur la face du moyen de maintien (2) tournée vers le support (1) une fermeture à crampons.

Fig. 1

Fig. 2

Fig. 3

Fig. 4

Fig. 5

Fig. 6

Fig. 7

Fig. 8